# EUROPEAN PATENT SPECIFICATION

(11) **EP 0 296 864 B1**
(45) Date of publication and mention of the grant of the patent: **15.09.1993**
(21) Application number: 88305754.9
(22) Date of filing: 24.06.1988
(51) Int. Cl.: C07D 285/135, C07D 277/82, C07D 285/06, C07D 285/08

(54) **Process for preparing ureas**
Verfahren zur Herstellung von Harnstoffen
Procédé pour la préparation d'urées.

(30) Priority: 25.06.1987 US 66916
(43) Date of publication of application: 28.12.1988
(73) Proprietor: DOWELANCO, Indianapolis, Indiana 46268-1189 (US)
(72) Inventor: Achgill, Ralph Kenneth, Lafayette Indiana 47905 (US); Call, Laurence Wesley, Lafayette indiana 47904 (US)
(74) Representative: Raynor, John

(56) References cited:
- CH-A- 554 886
- FR-A- 2 469 399
- US-A- 2 409 712
- JOURNAL OF THE CHEMICAL SOCIETY, CHEMICAL COMMUNICATIONS, no. 8, 23rd April 1969, page 414, London, GB; D.J. HAMILTON et al.: "Cleavage of amides with methanolic boron trifluoride"
- HOUBEN-WEYL, Methoden der organischen Chemie, vol. 8, 1952, pages 140,141, George Thieme Verlag, Stuttgart, DE; R. CRIEGEE et al.: "Sauerstoffverbindungen III"
- Fieser and Fieser, Reagents for organic Synthesis; New York, 1967, page 114, 115.

## Description

This invention provides a novel process for preparing herbicidal and insecticidal ureas.

Several methods of preparing ureas are disclosed for example in Houben-Weyl, volume E4, pages 178 and 343,344.

Thiadiazolyl and benzothiazolylureas are taught as herbicides in patents such as United States Patent No. 4,412,079 or 2,756,135 and British Patent No. 1,254,468.

The preparation of these herbicidal and insecticidal compounds generally requires the use of rather dangerous reactants such as phosgene or an isocyanate such as methylisocyanate. These reactants, especially the isocyanates, are well known as highly toxic substances, as evidenced by the recent industrial accident in India involving the release of methylisocyanate into the atmosphere, resulting in great injury and loss of human life.

According to the present invention there is provided a process for preparing compounds of the formula 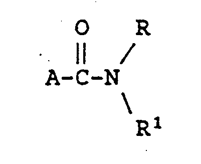
wherein A is 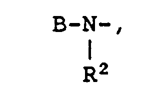
B is 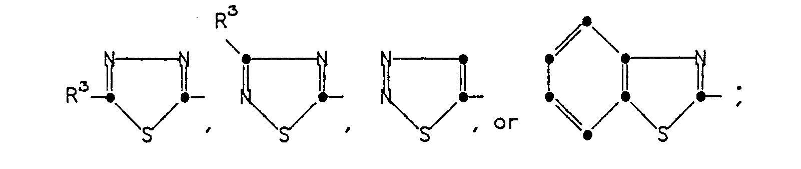
R is C₁-C₇ alkyl;
R¹ is H, C₁-C₇ alkyl, C₃-C₇ cycloalkyl, phenyl, or C₁-C₄ alkylphenyl;
R² is H, C₁-C₇ alkyl, C₃-C₇ cycloalkyl , C₂-C₇ alkenyl, C₂-C₇ alkynyl, or phenyl optionally substituted with Cl, Br, -NO₂, -CF₃, C₁-C₄ alkyl, C₁-C₄ alkoxy, or -N(C₁-C₄ alkyl)₂;
R³ is H, F, Cl, Br, -CF₃, C₁-C₇ alkyl optionally substituted with F, Cl, or Br, -S(O)ₙ C₁-C₇ alkyl, C₁-C₇ alkoxy, C₃-C₇ cycloalkyl optionally substituted with F, Cl, or Br, or phenyl optionally substituted with F, Cl, Br, -NO₂, -CF₃, or C₁-C₄ alkyl;
comprising reacting a nucleophile of the formula A-H, wherein A is as defined above, with a urea of the formula 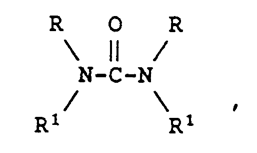
wherein each R is independently chosen from R as set forth above, and each R¹ is independently chosen from R¹ as set forth above.

Since the present invention provides a process for preparing herbicidal and insecticidal ureas by reacting a nucleophile, such as an amine, with a urea, it obviates the need to use dangerous reactants such as an isocyanate or phosgene.

A preferred group of compounds, which can be prepared by the present process are those wherein B is

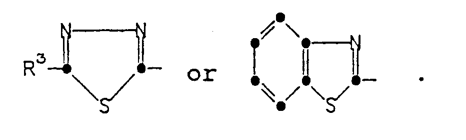

A more preferred group of compounds, wherein B is as above, which can be prepared by the present process are those wherein R is C₁-C₄ alkyl; R¹ is H or C₁-C₄ alkyl; R² is H, C₁-C₄ alkyl, or phenyl; and R³ is H, -CF₃, or C₁-C₄ alkyl. The most preferred compounds, wherein B is as above, which can be prepared by the present process are 1-(5-[t-butyl]-1,3,4-thiadiazol-2-yl)-1,3-dimethylurea (generally known as tebuthiuron), 1-(5-trifluoromethyl-1,3,4-thiadiazol-2-yl)-1,3-dimethylurea (generally known as thiazfluron) and 1-benzothiazol-2-yl-1,3-dimethylurea (generally known as methabenzthiazuron). Tebuthiuron, thiazfluron and methabenzthiazuron are all commercial herbicides.

The process of the present invention can be used to produce thiadiazolyl and benzothiazolylureas, such as those taught in references such as United States Patent Nos. 4,412,079, 2,756,135, 4,130,414, 4,174,398, and 3,917,478, British Patent Nos. 1,254,468 and 1,195,672, and Belgian Patent No. 743,614, from amine starting materials. The reader is referred to the above references with respect to the preparation of the starting materials, as well as the utility of the end-product ureas.

It is believed that one skilled in the art of organic chemistry would be aware of the types of ureas which can be prepared by the process of the present invention. However, the following specific compounds are mentioned in order to illustrate the type of herbicides and insecticides which can be produced by the process of this invention.
tebuthiuron
thiazfluron
benzthiazuron
methabenzthiazuron
All of the above are commercial herbicides or insecticides.

Certain of the compounds produced by the present process have substituents which may be optionally substituted with various groups as defined above. Optionally substituted, as used in this application, includes both mono- and poly-substitution, wherein each substituent is independently selected from the list of choices provided. All of such substituted compounds are well known in the art.

The claimed process may be conducted by reacting a nucleophile with a urea in an inert solvent at a temperature of from about 100° to about 200°C. Inert solvents are those in which the reactants are substantially soluble and which do not undergo chemical reaction during the process. Examples of typical inert solvents which can be used include alkanes and cycloalkanes such as cycloheptane, octane or decane; halogenated alkanes such as 1,1,2-trichloroethane, 1,2-bromoethane or bromoform; ethers such as 1,3-dioxane or diethylene glycol dimethyl ether; aromatic solvents such as toluene, chlorobenzene, the xylenes, cumene or o-dichlorobenzene; and aromatic heterocyclic solvents such as pyridine, pyrrole, the picolines or the lutidines. Preferred solvents employed in the process of the present invention are the aromatic solvents, especially the xylenes or cumene.

A nucleophile, as defined for purposes of the present invention, is an amine as defined by the formula A-H, wherein A is as previously delineated. The nucleophile starting materials are either commercially available, described in the literature in patents such as those cited previously, or can be prepared by methods known in the art. The concentration of nucleophile starting material in the solvent is not critical. In general, it is desirable to use as concentrated a solution as possible in order to minimize product loss during isolation. However, it is also desirable that enough solvent be used such that the product urea remains in solution throughout the reaction.

The urea starting materials employed are either commercially available, known in the literature, or can be prepared by methods known in the art. The amount of urea employed is not critical so long as at least an equimolar amount relative to the nucleophile starting material is used. Generally, it is preferred that a slight molar excess of the urea be used, e.g., from about 1.1 molar equivalents to about 3.0 molar equivalents, with 1.5 molar equivalents being most preferred.

The urea starting materials employed are of the formula 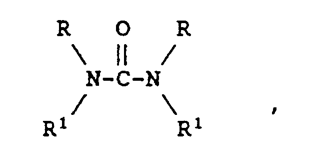
wherein each R and R¹ are independently chosen from the definition of R and R¹ set forth previously. Thus, the process of the invention can employ either a symmetrical or unsymmetrical urea as starting material. Examples of typical symmetrical or unsymmetrical ureas which can be employed in the process of the invention include such compounds as 1,3-dimethylurea, 1,3-diethylurea, 1,3-dipropylurea, 1,3-dimethyl-1,3-dipropylurea, 1,1-dipropyl-3-methylurea, 1,1-dibutyl-3-ethylurea, 1,3-dimethyl-1-cyclopentylurea, 1-methyl-1-methylphenyl-3-pentylurea, 1,3-diethyl-1-methyl-3-phenylurea, 1,1-dihexyl-3,3-diethylurea and 1-ethyl-1-phenyl-3-butyl-3-methylurea.

If a tetra-substituted unsymmetrical urea (i.e. none of the R¹ groups is H) is employed, the reaction product will be a mixture of two compounds. For example, if 1,1-dipropyl-3,3-dimethylurea is reacted with a nucleophile of the formula A-H the products produced will be 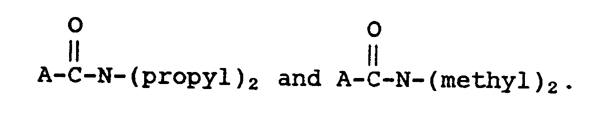
The desired product, for instance 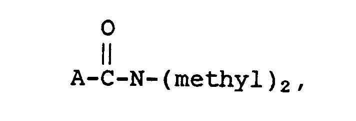
can then be isolated from the less desired compound by using standard isolation techniques such as distillation or crystallization.

To avoid the yield loss and isolation problems associated with the tetra-substituted unsymmetrical urea, a preferred process of the present invention uses either a tri-substituted unsymmetrical urea (one of the R¹ groups is H) or a symmetrical urea (both R groups the same, both R¹ groups the same) as starting material. It has been discovered that when a tri-substituted unsymmetrical urea is used as starting material, compounds of the formula 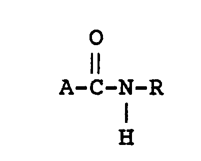
are preferentially produced relative to those of the formula 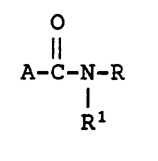
(R¹ is not H). Thus, products of the formula 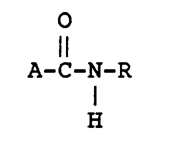
can be prepared in high yield and without expensive purification steps when tri-substituted unsymmetrical urea starting materials are employed.

Compounds of the formula 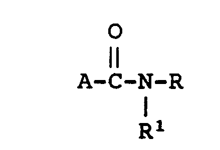
can also be prepared in high yield and without expensive purification steps using a symmetrical urea starting material. Symmetrical ureas are equally useful for preparing compounds where R¹ is H and compounds where R¹ is a substituent selected from the group consisting of C₁-C₇ alkyl, C₃-C₇ cycloalkyl, phenyl, or C₁-C₄ alkylphenyl, since either side of the urea molecule will produce the same final product. Processes of the present invention which use a symmetrical urea starting material to prepare urea final products are especially preferred.

An acid, while not required by the present process, can be employed to increase reaction between the nucleophile and the urea. Suitable acids which aid the present reaction include inorganic acids such as hydrochloric acid or hydrobromic acid and organic acids such as methanesulfonic acid or p-toluene-sulfonic acid. Inorganic acids are preferred, especially hydrochloric acid, which can be used as either an anhydrous gas or as a concentrated aqueous solution. The amount of acid employed is not critical and will generally range from about 1.0 molar equivalents to about 3.0 molar equivalents relative to the nucleophile starting material. A preferred amount is about 1.5 molar equivalents.

If an organic or inorganic acid is to be employed in a process which requires an amine nucleophile starting material, a molar equivalent of the acid may be added by forming and isolating an acid addition salt of the amine starting material prior to use in the process of the invention. The isolated salt, mixed with a symmetrical or unsymmetrical urea in an inert solvent as described above for the non-salt compounds, provides both the required amine nucleophile starting material as well as one molar equivalent of acid. Additional acid may be added as desired if greater than an equimolar amount relative to the amine nucleophile is desired.

The process generally is conducted at reflux, for example at a temperature of about 100 °C to about 200°C. The process is substantially complete after about 1 to about 48 hours when conducted at the solvent's reflux temperature. The progress of the reaction can be followed, if desired, by standard high performance liquid chromatography (HPLC) analytical techniques in order to determine when the reaction is substantially complete.

Once the reaction is substantially complete, the urea product can be isolated, if desired, by first cooling the solution to below 100°C and adding water in order to remove unreacted urea starting material and any acid. The resulting aqueous layer may be separated from the organic layer, if desired, but need not be, and the resulting mixture cooled to about 0°C. The product compounds generally crystallize and can be isolated by standard filtration techniques in good purity and high yield.

The filtrate and the above described aqueous layer both often contain unreacted nucleophile starting material, as well as some urea final product. These compounds can be recovered, if desired, by combining the filtrate and the aqueous layer and separating the resulting aqueous layer from the organic layer. The recovered organic layer is then combined with fresh solvent and additional nucleophile starting material, and the process of the present invention is repeated with the recycled material.

The following Examples illustrate specific aspects of the present invention.

### Preparation 1

### 2-(t-Butyl)-5-methylamine-1,3,4-thiadiazole hydrochloride

To a one liter, 3-neck flask, with an agitator and a sub-surface gas addition system, were added 425 ml of a toluene solution which contained 250 g (1.46 mol) of 2-(t-butyl)-5-methylamine-1,3,4-thiadiazole. The solution was diluted by adding 325 ml of reagent grade toluene and heated to about 60°C. Anhydrous hydrochloric acid gas (60.0 g, 1.63 mol) was added at a rate sufficient to keep the solution temperature between 60°C and 70°C. The resulting slurry was cooled to 0°C and stirred for about 30 minutes at that temperature. The precipitated solid, recovered by filtration, was washed with 250 ml of toluene and dried overnight in a vacuum oven at 60°C to provide 310.5 g (97.7% yield) of 2-(t-butyl)-5-methylamine-1,3,4-thiadiazole hydrochloride.

### Preparation 2

### 1,1-Dipropyl-3-methylurea

To a 250 ml, 3-neck flask, with an agitator and condenser were added 22.0 g (0.25 mol) of 1,3-dimethylurea, 25.3 g (0.25 mol) of dipropylamine and 100 ml of cumene. The solution was heated at reflux (about 137°C) for about 12 hours, cooled to 100°C, and the solvent was removed using vacuum distillation to provide a residue. The residue was dried in a vacuum oven at 60°C to provide 41.5 g of 1,1-dipropyl-3-methylurea.

### Example 1

### 1-(5-[t-Butyl]-1,3,4-thiadiazole-2-yl)-1,3-dimethylurea

To a 250 ml, 3-neck flask, equipped with an agitator were added 34.4 g of 74.7% pure 2-(t-butyl)-5-methylamine-1,3,4-thiadiazole (0.15 moles of the amine), 30 ml of deionized water, 25 ml of xylenes (a reagent grade mixture of o, m, and p xylene), and 20.0 g of a 34.0% by weight hydrochloric acid solution (0.18 moles of hydrochloric acid). The mixture was heated to about 55°C, stirred for 15 minutes, and the organic layer separated from the aqueous layer.

The aqueous layer was mixed with 75 ml of xylenes. Sodium hydroxide (15.4 g of 50% by weight sodium hydroxide solution) was added until the mixture's pH was about 8.0. The resulting two-phase solution was heated to about 50°C, stirred for 15 minutes, and the organic layer separated from the aqueous layer. The aqueous layer was extracted with an additional 30 ml of xylenes.

The organic extract was combined with the above organic layer and both solutions were added to a 250 ml, 3-neck flask, equipped with an agitator, condenser and a Dean Stark trap. The solution was dried by distilling an azeotropic mixture of water and xylenes until the solution's temperature reached about 145°C.

Once the solution was dried it was cooled to about 50°C and 15.9 g (0.18 mol) of 1,3-dimethylurea were added. Anhydrous hydrochloric acid gas (6.6 g, 0.18 mol) was added sub-surface to the liquid. The resulting solution was heated until the xylenes solvent began to reflux (about 142°C) and stirred at that temperature for 10 hours. After 10 hours the solution was cooled to about 70°C and 30 ml of deionized water were added. The resulting two-phase mixture was stirred for 15 minutes and then the organic layer was separated from the aqueous layer.

The organic layer was mixed with 30 ml of deionized water and cooled to 0°C. After stirring for 1 hour at 0°C the resulting slurry was filtered and the recovered crystals washed with 25 ml of deionized water. These crystals were dried in a vacuum oven at 60°C to provide 24.6 g (70.1% yield) of 97.4% pure 1-(5-[t-butyl]-1,3,4-thiadiazol-2-yl)-1,3-dimethylurea. m.p. = 161.0°-162.0°C.

The filtrate from the above filtration was combined with the reaction solution aqueous wash noted above and the pH of the resulting mixture was adjusted to 8.0 using a 50% by weight sodium hydroxide solution. The organic layer was separated from the aqueous layer and placed into a 100 ml volumetric flask. Additional xylenes solvent was added to the volumetric flask until the liquid volume reached 100 ml. One milliliter was withdrawn from the volumetric flask and mixed with acetonitrile until the resulting solution's volume was 100 ml. This solution was assayed by high performance liquid chromatography (HPLC) which disclosed that the filtrate and aqueous layers contained a total of 1.6 g of product and 2.9 g of unreacted amine starting material.

### Example 2

### 1-(5-[t-Butyl]-1,3,4-thiadiazole-2-yl)-1,3-dimethylurea

To a 250 ml, 3-neck flask, equipped with an agitator were added 34.4 g of 74.7% pure 2-(t-butyl)-5-methylamine-1,3,4-thiadiazole (0.15 moles of the amine), 40 ml of deionized water, and 75 ml of xylenes. The pH of the mixture was adjusted to about 8.0 by adding 1.6 g of a 50% by weight sodium hydroxide solution. The basic two-phase solution was heated to about 50°C and stirred at that temperature for 15 minutes. The aqueous layer was separated from the organic layer and extracted with an additional 30 ml of xylenes.

The extract was combined with the above organic layer and both were added to a 250 ml, 3-neck flask, equipped with an agitator, condenser, and a Dean Stark trap. The solution was dried by distilling an azeotropic mixture of water and xylenes as in Example 1.

Once the solution was dried it was cooled to about 50°C and 15.9 g (0.18 mol) of 1,3-dimethylurea were added. Anhydrous hydrochloric acid gas (6.6 g, 0.18 mol) was added sub-surface to the liquid. The resulting solution was reacted and washed according to the procedure described in Example 1. The organic layer, mixed with water, cooled, filtered, and dried in an analogous manner to the organic layer described in Example 1, provided 20.3 g (58.2% yield) of 98.0% pure 1-(5-[t-butyl]-1,3,4-thiadiazol-2-yl)-1,3-dimethylurea. m.p. = 161.0°-162.0°C.

The filtrate and the reaction solution aqueous wash were combined and the pH of the resulting mixture was adjusted to 8.0 using a 50% by weight sodium hydroxide solution. The resulting organic layer was separated and diluted according to the procedure of Example 1. High performance liquid chromatography (HPLC) assay of the final solution disclosed that the filtrate and aqueous layers contained a total of 2.2 g of product and 4.2 g of unreacted starting material.

### Example 3

### 1-(5-[t-Butyl]-1,3,4-thiadiazole-2-yl)-1,3-dimethylurea

To a 500 ml, 3-neck flask, equipped with an agitator and a condenser were added 31.0 g (0.15 mol) of 2-(t-butyl)-5-methylamine-1,3,4-thiadiazole hydrochloride prepared according to the procedure of Preparation I, 20.0 g (0.23 mol) of 1,3-dimethylurea, and 75 ml of cumene. Additional hydrochloric acid was added by introducing anhydrous hydrochloric acid gas (3.0 g, 0.08 mol) sub-surface to the liquid. The resulting solution was heated until the solvent began to reflux (about 150°C) and stirred at that temperature for about 6 hours. After 6 hours the solution was cooled to 80°C and 30 ml of deionized water were added. The resulting two-phase mixture was stirred for 15 minutes and then the organic layer separated from the aqueous layer.

The organic layer was mixed with 30 ml of deionized water and cooled to 0°C. After stirring the mixture for 30 minutes at a temperature of about 0°C the resulting slurry was filtered and the recovered crystals washed with 30 ml of deionized water. This material, dried in a vacuum oven at 60°C, provided 28.8 g (83.5% yield) of 99.2% pure 1-(5-t-butyl]-1,3,4-thiadiazol-2-yl)-1,3-dimethylurea. m.p. = 160.8°-163.6°C.

The filtrate and the aqueous layer from above were combined and the resulting mixture treated according to the procedures described in Example 1. HPLC assay of the dilute solution indicated that the mixture contained 1.2 g of product and 1.8 g of unreacted starting material.

### Example 4

### 1-(5-[t-Butyl]-1,3,4-thiadiazole-2-yl)-1,3-dimethylurea

The procedure of Example 3 was repeated with the exception that the reaction solution was heated until the solvent began to reflux and stirred at that temperature for about 4 hours. This reaction time provided 28.3 g (82.4% yield) of a 99.6% pure 1-(5-[t-butyl]-1,3,4-thiadiazol-2-yl)-1,3-dimethylurea product (m.p. 160.6°-162.8°C) and a filtrate/aqueous layer mixture which contained 1.1 g of product and 1.8 g of unreacted starting material.

### Example 5

### 1-(5-[t-Butyl]-1,3,4-thiadiazole-2-yl)-1,3-dimethylurea

To a 250 ml, 3-neck flask, equipped with an agitator, a condenser, and a Dean Stark trap were added 36.4 g (0.17 mol) of 2-(t-butyl)-5-methylamine-1,3,4-thiadiazole hydrochloride prepared according to the procedure of Preparation 1, 29.4 g (0.33 mol) of 1,3-dimethylurea, and 90 ml of xylenes. Seventeen grams of a 38.0% by weight aqueous hydrochloric acid solution (0.17 moles of hydrochloric acid) were added. The resulting mixture was dried by distilling an azeotropic mixture of xylenes and water until the solution's temperature reached about 135°C.

Once the solution was dried the solvent was refluxed rather than removed and the solution was stirred at the reflux temperature for about 16 hours. The solution was then cooled to about 75°C and 25 ml of deionized water were added. The resulting two-phase mixture was stirred for 15 minutes and the organic layer separated from the aqueous layer. Deionized water (25 ml) was used to wash the organic phase a second time.

The organic layer was separated from the second aqueous wash layer and 25 ml of deionized water were added. The mixture was cooled to about 0°C and stirred at that temperature for 30 minutes. The resulting slurry was filtered and the recovered product was washed with 40 ml of deionized water and dried in a vacuum oven at 60°C to provide 27.6 g (71.6% yield) of 98.6% pure 1-(5-[t-butyl]-1,3,4-thiadiazol-2-yl)-1,3-dimethylurea. m.p. 161.0°-162.8°C.

The filtrate and aqueous layers from above were combined and the resulting mixture treated according to the procedures of Example 1. HPLC assay of the dilute solution indicated that the mixture contained 2.0 g of product urea and 1.8 g of unreacted starting material.

### Example 6

### 1-(5-[t-Butyl]-1,3,4-thiadiazole-2-yl)-1,3-dimethylurea

The procedure of Example 1 was repeated with the exceptions that methanesulfonic acid (15.4 g of Aldrich reagent grade 98.0% by weight methanesulfonic acid solution, 0.16 mol of acid) was used in place of hydrochloric acid and the solution was heated until the solvent began to reflux and stirred at that temperature for five hours. This process provided 18.9 g (53.8% yield) of 97.4% pure 1-(5-[t-butyl]-1,3,4-thiadiazole-2-yl)-1,3-dimethylurea product (m.p. 161.0°-162.6°C) and a filtrate/aqueous layer mixture which contained 3.5 g of product and 5.4 g of unreacted starting compound.

### Example 7

### 1-(5-[t-Butyl]-1,3,4-thiadiazole-2-yl)-1,3-dimethylurea

To a 250 ml, 3-neck flask, equipped with an agitator and a condenser were added 35.0 g (0.17 mol) of 2-(t-butyl)-5-methylamine-1,3,4-thiadiazole hydrochloride prepared according to the procedure of Preparation 1, 16.2 g (0.18 mol) of 1,3-dimethylurea, 100 ml of xylenes, and 16.8 g (0.17 mol) of triethylamine. Anhydrous hydrochloric acid gas (7.4 g, 0.20 mol) was added sub-surface to the liquid at a rate which kept the liquid temperature below 70°C. The resulting solution was heated until the solvent began to reflux (about 142°C) and stirred at that temperature for 10 hours. After 10 hours the solution was cooled to 80°C and 25 ml of deionized water were added. The resulting two-phase mixture was stirred for 15 minutes and the organic layer separated from the aqueous layer.

The organic layer was mixed with 25 ml of deionized water and cooled to about 0°C, then stirred at that temperature for 30 minutes. The resulting slurry was filtered and the recovered crystals washed with 30 ml of water then dried in a vacuum oven at 60°C to provide 25.3 g (66.5% yield) of 99.4% pure 1-(5-[t-butyl]-1,3,4-thiadiazole-2-yl)-1,3-dimethylurea. m.p. 161.0°-162.4°C.

The filtrate and aqueous layer above were combined and the resulting mixture was treated according to the procedures described in Example 1. HPLC assay of the dilute solution disclosed that the mixture contained 2.9 g of urea product and 5.4 g of unreacted starting material.

### Example 8

### 1-(5-[t-Butyl]-1,3,4-thiadiazole-2-yl)-1,3-dimethylurea

The title compound was prepared from 1,1-dipropyl-3-methylurea in the following manner. 1,1-Dipropyl-3-methylurea was prepared by adding 150 ml of cumene and 25.3 g (0.25 mol) of dipropylamine to a 250 ml, 3-neck flask, equipped with an agitator and a dropping funnel. Methylisocyanate (16.0 g, 0.28 mol) was added at a rate such that the temperature of the contents of the flask remained below 60°C. Once methylisocyanate addition was complete, the resulting solution was heated to about 60°C and stirred at that temperature for 30 minutes. The solution was then heated to reflux (150°C) and a total of 20 ml of cumene were distilled to remove the unreacted methylisocyanate.

The reaction mixture was then cooled to about 100°C and 51.9 g (0.25 mol) of 2-(t-butyl)-5-methylamine-1,3,4-thiadiazole hydrochloride (prepared according to the procedure of Preparation 1) were added. The resultsolution was heated to reflux (about 154°C) for about 2 hours, cooled to 85°C, and diluted by the addition of 50 ml of deionized water. The resulting two-phase mixture was stirred for 30 minutes at 80°C, and then the organic layer separated from the aqueous layer.

The organic layer was mixed with 50 ml of fresh deionized water, cooled to about 0°C, and stirred at that temperature for 30 minutes. The resulting slurry was filtered and the recovered crystals washed with 50 ml of water, then dried in a vacuum oven at 60°C to provide 43.8 g (75.5% yield) of 98.2% pure 1-(5-[t-butyl]-1,3,4-thiadiazole-2-yl)-1,3-dimethylurea. mp = 160.4°-162.4°C. The product wad identified by n.m.r. analysis (60 mHz, CDCl₃): δ = 1.55 (singlet, 9H); 3.10 (doublet, 3H); 3.65 (singlet, 3H); 8.05 (broad singlet, 1H).

The filtrate and aqueous layer above were combined and the resulting mixture treated according to the procedures described in Example 1. HPLC assay of the dilute solution disclosed that the mixture contained 2.4 g of urea product and 3.8 g of unreacted starting material.

### Example 9

### 1-(5-[t-butyl]-1,3,4-thiadiazole-2-yl)-1-methly-3-ethylurea

To a 250 ml, 3-neck flask, equipped with an agitator and a condenser were added 21.0 g (0.10 mol) of 2-(t-butyl)-5-methylamine-1,3,4-thiadiazole hydrochloride, 13.9 g (0.14 mol) of 1,3-diethylurea, and 60 ml of cumene. Additional hydrochloric acid was added by introducing anhydrous hydrochloric acid gas (1.8 g, 0.05 mol) sub-surface to the liquid. The resulting solution was heated to reflux (about 150°C) and stirred at that temperature for about 6 hours. After 6 hours the solution was cooled to 85°C and diluted with 30 ml of deionized water. The resulting two-phase mixture was stirred for 30 minutes at 80°C and then the organic layer separated from the aqueous layer.

The organic layer was mixed with 30 ml of deionized water and the pH of the resulting mixture was lowered to about 1.0 by adding a few drops of concentrated hydrochloric acid (38% by weight hydrochloric acid in water), while stirring. Again, the organic layer was separated from the aqueous layer. The organic layer was concentrated to an oily residue under reduced pressure. The oily product, stored in a vacuum oven at 70°C overnight, was cooled to room temperature (22°C) and allowed to solidify over the next three days to provide 12.1 g of solid product. The solid product was identified as 1-(5-[t-butyl]-1,3,4-thiadiazole-2-yl)-1-methyl-3-ethylurea by comparing n.m.r. spectra with a 1-(5-[t-butyl]-1,3,4-thiadiazole-2-yl)-1-methyl-3-ethylurea reference standard. The n.m.r. analyses were conducted on a 60 mHz instrument in CDCl₃: δ = 1.25 (triplet, 3H); 1.50 (singlet, 9H); 3.65 (singlet, 3H); 7.20 (triplet, 2H); 7.75-8.20 (broad triplet,1H).

### Example 10

### 1-Benzothiazol-2-yl-1,3-dimethylurea

To a 250 ml, 3-neck flask, equipped with an agitator, a condenser, and a sub-surface gas addition system were added 16.4 g (0.10 mol) of 2-methylaminobenzothiazole, 13.2 g (0.15 mol) of 1,3-dimethylurea and 100 ml of cumene. Anhydrous hydrochloric acid gas (5.5 g, 0.15 mol) was added sub-surface to the liquid at a rate which kept the liquid temperature below 90°C. The resulting solution was heated to reflux (about 150°C) and stirred at that temperature for about 6 hours. After 6 hours the solution was cooled to about 90°C and 50 ml of deionized water were added. The resulting two-phase mixture was stirred for 30 minutes at about 80°C and then the organic layer was separated from the aqueous layer.

The organic layer was mixed with 50 ml of deionized water and the resulting two-phase mixture heated to about 80°C. Several drops of 50% sodium hydroxide solution were added in order to increase the pH of the aqueous layer to about 11.0. The basic mixture was stirred for an additional five minutes and then the organic layer was again separated from the aqueous layer.

The organic layer was mixed, once more, with 50 ml of deionized water and the resulting mixture cooled to about 0°C. After stirring the mixture for 30 minutes at a temperature of about 0°C the resulting slurry was filtered and the recovered crystals washed with 25 ml of deionized water. This material, dried in a vacuum oven at 60°C, provided 12.2 g (55.2% yield) of 1-benzothiazol-2-yl-1,3-dimethylurea. mp = 120°-121°C. The product was identified as 1-benzothiazol-2-yl-1,3-dimethylurea by comparing n.m.r. spectra with a 1-benzothiazol-2-yl-1,3-dimethylurea reference standard. The n.m.r. analyses were conducted on a 60 mHz instrument in CDCl₃: δ = 3.10 (doublet, 3H); 3.60 (singlet, 3H); 7.25-7.65 (broad multiplet, 4H); 7.80 (doublet, 1H).

## Claims

1. A process for preparing a compound of the formula 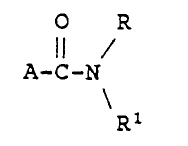 wherein A is 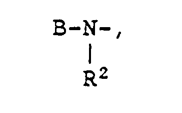 B is 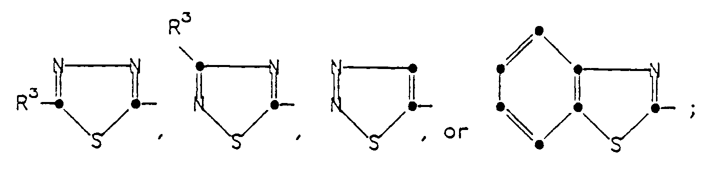 R is C₁-C₇ alkyl;
R¹ is H, C₁-C₇ alkyl, C₃-C₇ cycloalkyl, phenyl, or C₁-C₄ alkylphenyl;
R² is H, C₁-C₇ alkyl, C₃-C₇ cycloalkyl, C₂-C₇ alkenyl, C₂-C₇ alkynyl, or phenyl optionally substituted with Cl, Br, -NO₂, -CF₃, C₁-C₄ alkyl, C₁-C₄ alkoxy, or -N(C₁-C₄ alkyl)₂;
R³ is H, F, Cl, Br, -CF₃, C₁-C₇ alkyl optionally substituted with F, Cl, or Br, -S(O)ₙ C₁-C₇ alkyl, C₁-C₇ alkoxy, C₃-C₇ cycloalkyl optionally substituted with F, Cl, or Br, or phenyl optionally substituted with F, Cl, Br, -NO₂, -CF₃, or C₁-C₄ alkyl; and n is 0,1 or 2. comprising reacting a nucleophile of the formula A-H, wherein A is as defined above, with a urea of the formula 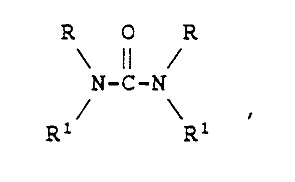 wherein each R is independently chosen from R as set forth above and each R¹ is independently chosen from R¹ as set forth above.

2. A process of Claim 1 in which the urea is a tri-substituted unsymmetrical urea.

3. A process of Claim 1 in which the urea is a symmetrical urea.

4. A process of any one of Claims 1, 2 or 3 wherein
B is 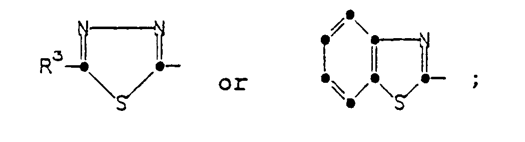 R is C₁-C₄ alkyl; R¹ is H or C₁-C₄ alkyl; R² is H, C₁-C₄ alkyl or phenyl; and R³ is H, -CF₃ or C₁-C₄ alkyl.

5. A process of Claim 4 wherein the product urea is 1-(5-[t-butyl]-1,3,4-thiadiazole-2-yl)-1,3-dimethylurea.

6. A process of any one of Claims 1 to 5 in which an inert solvent is used.

7. A process of any one of Claims 1 to 6 in which an organic or inorganic acid is used to increase reaction between the nucleophile and the urea.

## Patentansprüche

1. Verfahren zur Herstellung einer Verbindung der Formel 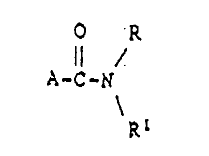 worin A 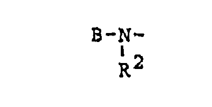 ist,
B 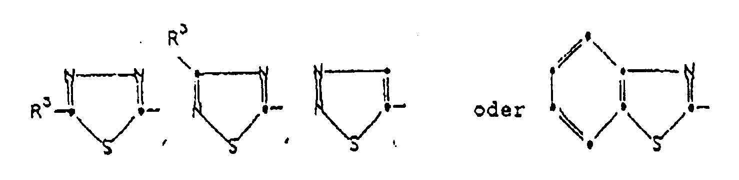 ist,
R C₁-C₇-Alkyl ist,
R¹ H, C₁-C₇-Alkyl, C₃-C₇-Cycloalkyl, Phenyl oder C₁-C₄-Alkylphenyl ist,
R² H, C₁-C₇-Alkyl, C₃-C₇-Cycloalkyl, C₂-C₇-Alkenyl, C₂-C₇-Alkinyl oder gegebenenfalls mit Cl, Br, -NO₂, -CF₃, C₁-C₄-Alkyl, C₁-C₄-Alkoxy oder -N(C₁-C₄-Alkyl)₂ substituiertes Phenyl ist,
R³ H, F, Cl, Br, -CF₃, gegebenenfalls mit F, Cl oder Br substituiertes C₁-C₇-Alkyl, -S(O)ₙC₁-C₇-Alkyl, C₁-C₇-Alkoxy, gegebenenfalls mit F, Cl oder Br substituiertes C₃-C₇-Cycloalkyl oder gegebenenfalls mit F, Cl, Br, -NO₂, -CF₃) oder C₁-C₄-Alkyl substituiertes Phenyl ist, und n 0, 1 oder 2 ist
umfassend die Reaktion eines Nukleophils der Formel A-H, worin A wie oben definiert ist, mit einem Harnstoff der Formel 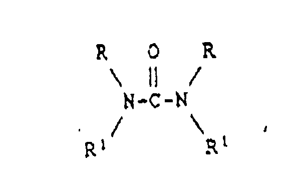 worin jedes R unabhängig aus R wie oben angegeben ausgewählt ist und jedes R¹ unabhängig aus R¹ wie oben angegeben ausgewählt ist.

2. Verfahren nach Anspruch 1, worin der Harnstoff ein dreifach substituierter asymmetrischer Harnstoff ist.

3. Verfahren nach Anspruch 1, worin der Harnstoff ein symmetrischer Harnstoff ist.

4. Verfahren nach einem der Ansprüche 1,2 oder 3, worin
B 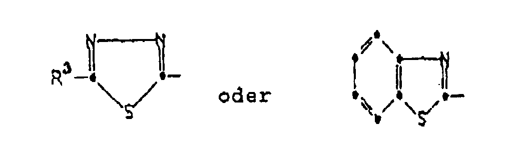 ist,
R C₁-C₄-Alkyl ist, R¹ H oder C₁-C₄-Alkyl ist, R² H, C₁-C₄-Alkyl oder Phenyl ist und R³ H, CF₃ oder C₁-C₄-Alkyl ist.

5. Verfahren nach Anspruch 4, worin der Produktharnstoff 1-(5-[t-Butyl]-1,3,4-thiadiazol-2-yl)-1,3-dimethylharnstoff ist.

6. Verfahren nach einem der Ansprüche 1 bis 5, worin ein inertes Lösungsmittel verwendet wird.

7. Verfahren nach einem der Ansprüche 1 bis 6, worin eine organische oder anorganische Säure zur Steigerung der Reaktion zwischen dem Nukleophil und dem Harnstoff verwendet wird.

## Revendications

1. Procédé de préparation d'un composé de formule 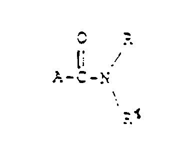 dans laquelle A représente B-N-R²;
B représente 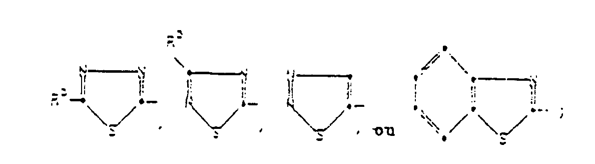 R représente un groupe alkyle en C₁-C₇ ;
R¹ représente un atome d'hydrogène ou un groupe alkyle en C₁-C₇, cycloalkyle en C₃-C₇, phényle ou (alkyle en C₁-C₄)-phényle ;
R² représente un atome d'hydrogène ou un groupe alkyle en C₁-C₇, cycloalkyle en C₃-C₇, alcényle en C₂-C₇, alcynyle en C₂-C₇, ou phényle éventuellement substitué par -Cl, -Br, -NO₂, -CF₃, alkyle en C₁-C₄, alkoxy en C₁-C₄ ou -N(alkyle en C₁-C₄)₂ ;
R³ représente un atome H, F, Cl ou Br, ou un groupe -CF₃, alkyle en C₁-C₇ éventuellement substitué par -F, -Cl ou -Br, -S(O)ₙ-(alkyle en C₁-C₇), alkoxy en C₁-C₇, cycloalkyle en C₃-C₇ éventuellement substitué par -F, -Cl ou -Br, ou phényle éventuellement substitué par -F, -Cl, -Br, -NO₂, -CF₃ ou alkyle en C₁-C₄ ;
et n vaut 0, 1 ou 2;
ledit procédé comprenant la réaction d'un nucléophile de formule A-H, dans laquelle A est tel que défini plus haut, avec une urée de formule 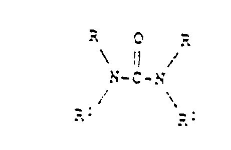 dans laquelle chaque R est indépendamment choisi parmi les R indiqués ci-dessus et chaque R¹ est indépendamment choisi parmi les R¹ indiqués ci-dessus.

2. Procédé selon la revendication 1, dans lequel l'urée est une urée trisubstituée non-symétrique.

3. Procédé selon la revendication 1, dans lequel l'urée est une urée symétrique.

4. Procédé selon l'une des revendications 1, 2 ou 3, dans lequel B représente 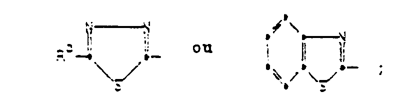 R représente un groupe alkyle en C₁-C₄, R¹ représente un atome d'hydrogène ou un groupe alkyle en C₁-C₄, R² représente un atome d'hydrogène ou un groupe alkyle en C₁-C₄ ou phényle et R³ représente un atome d'hydrogène ou un groupe -CF₃ ou alkyle en C₁-C₄.

5. Procédé selon la revendication 4, dans lequel l'urée produite est la 1-(5-tertiobutyl-1,3,4-thiadiazole-2-yl)-1,3-diméthyl-urée.

6. Procédé selon l'une des revendications 1 à 5, dans lequel on utilise un solvant inerte.

7. Procédé selon l'une des revendications 1 à 6, dans lequel on utilise un acide organique ou minéral pour promouvoir la réaction entre le nucléophile et l'urée.
